# FASCICULE DE BREVET EUROPEEN

(11) **EP 0 811 632 B1**
(45) Date de publication et mention de la délivrance du brevet: **08.08.2001**
(21) Numéro de dépôt: 97401220.5
(22) Date de dépôt: 02.06.1997
(51) Int. Cl.: C07H 7/027

(54) **Procédé de décarboxylation des acides 2-cétoaldoniques**
Verfahren zur Decarboxylierung von 2-Ketoaldonisch-Säuren
Process of decarboxylation of 2-ketoaldonic acids

(30) Priorité: 03.06.1996 FR 9606808
(43) Date de publication de la demande: 10.12.1997
(73) Titulaire: ROQUETTE FRERES, 62136 Lestrem (FR)
(72) Inventeur: Fleche, Guy, 59190 Hazebrouck (FR); Duflot, Pierrick, 62136 Lacouture (FR)
(74) Mandataire: Boulinguiez, Didier

(56) Documents cités:
- EP-A- 0 716 067
- WO-A-93/19030
- AGR. BIOL. CHEM., vol. 27, no. 3, 1963, pages 180-184, XP000617203 M.MATSUI ET AL.: "Studies on 2-Keto-D-gluconic Acid. Part I. Metal Ion Catalysed Decarboxylation of 2-Keto-aldonic Acid."

## Description

La présente invention a pour objet un procédé particulier de décarboxylation des acides 2-cétoaldoniques.

Plus précisément, la présente invention a pour objet un procédé de décarboxylation des acides 2-cétoaldoniques catalysée par les ions nickel, et se déroulant en phase aqueuse.

Le procédé de la présente invention permet d'obtenir avec un excellent rendement le cétose de rang immédiatement inférieur correspondant à cet acide aldonique.

C'est ainsi que le procédé de la présente invention permet d'obtenir, par exemple,
- le D-ribulose à partir de l'acide 2-céto-D-gluconique,
- le D-xylulose à partir de l'acide 2-céto-D-galactonique,
- le D-érythrulose à partir de l'acide 2-céto-D-arabinonique.

Ces cétoses, rares dans la nature, sont d'un grand intérêt en tant que tels, mais seraient surtout des intermédiaires de synthèse fort importants s'il advenait que l'on puisse les produire en grande quantité et à faible coût. En effet, des étapes complémentaires simples d'hydrogénation et/ou d'isomérisation de ces cétoses permettent d'obtenir facilement le xylitol, le D-arabitol, le D-ribitol, le D-threitol ou l'érythritol qui sont tous des polyols pouvant être employés dans de multiples applications et notamment en tant que substituts non cariogènes et hypocaloriques du saccharose.

Il existe un procédé de décarboxylation de certains acides 2-cétoaldoniques qui fournit avec d'excellents rendements les cétoses de rang immédiatement inférieur correspondant à ces acides.

Ce procédé, décrit par MATSUI et ses collaborateurs dans Agr. Biol. Chem., Vol. 27, N° 3, P 180 à 184, 1963, consiste à soumettre à l'action catalytique décarboxylante des ions Ni⁺⁺' certains acides 2-cétoaldoniques dissous dans de la pyridine anhydre chaude.

Ainsi, ces auteurs ont montré que l'acide 2-céto-D-gluconique fournissait le D-ribulose et un peu de son isomère le D-arabinose et que l'acide 2-céto-L-gulonique fournissait le L-xylulose accompagné de moindres quantités de son isomère, le L-xylose.

Il est probable que dans ce procédé, le caractère de base faible de la pyridine soit responsable de l'apparition de l'arabinose et du xylose par l'intermédiaire d'une isomérisation alcaline du ribulose ou du xylulose formés.

Un tel procédé, efficace et mettant en oeuvre des acides 2-cétoaldoniques qui s'obtiennent très aisément à partir des aldoses correspondants, très abondants et bon marché, n'a cependant jamais trouvé sa place dans l'industrie, notamment alimentaire.

En effet, il met en oeuvre de la pyridine, solvant extrêmement toxique et inflammable qu'il convient de surcroît d'employer à l'état anhydre et au voisinage de son point d'ébullition.

En outre, l'apparition par ce procédé, de produits d'isomérisation des cétoses peut dans certains cas s'avérer gênante.

Il existait donc un besoin de mettre au point un procédé performant de décarboxylation des acides 2-cétoaldoniques ne présentant pas les inconvénients rédhibitoires de l'art antérieur et qui soit à même de fournir avec d'excellents rendements et à l'état de haute pureté les cétoses de rang immédiatement inférieur correspondant à ces acides.

Et la Société Demanderesse a trouvé que lorsque l'on mettait une solution aqueuse d'un acide 2-cétoaldonique et de sels de nickel au contact d'une résine porteuse de groupements vinylpyridine, on obtenait avec un excellent rendement et à l'état de haute pureté le cétose de rang immédiatement inférieur correspondant à cet acide.

Selon l'invention, le procédé de décarboxylation catalytique des acides 2-cétoaldoniques par les ions nickel est donc caractérisé par le fait qu'une solution aqueuse d'un acide 2-cétoaldonique est mise au contact d'une résine porteuse de groupements vinylpyridine.

Le premier avantage d'un tel procédé par rapport au procédé de l'art antérieur est évident tant au plan de la toxicité qu'au plan de la sécurité puisqu'il ne met pas en oeuvre de pyridine chaude.

Un deuxième avantage du procédé selon l'invention réside dans le fait qu'il est extrêmement facile à mettre en oeuvre puisque la résine peut être séparée de la phase aqueuse réactionnelle par simple filtration et sans les précautions particulières de confinement et d'élimination que requerrait l'emploi d'un solvant organique aussi dangereux que la pyridine.

Un troisième avantage du procédé de l'invention est que le cétose est obtenu sans isomérisation parasite et avec un rendement quasi-stoechiométrique.

De préférence, le procédé de l'invention est mis en oeuvre à une température comprise entre 40° et 100°C. Des températures inférieures amènent des temps de réaction trop longs et des températures supérieures, outre qu'elles nécessiteraient l'emploi de réacteurs résistants à la pression, conduiraient soit à une dégradation progressive de la résine, soit à une dégradation des produits de la réaction. Les températures de 70° à 90°C sont particulièrement préférées dans le procédé de l'invention.

De préférence encore, le procédé de l'invention est mis en oeuvre avec des solutions aqueuses d'acides 2-cétoaldoniques dont la concentration est supérieure à 50g/litre, ceci pour des raisons évidentes d'économie d'évaporation de l'eau et de réduction de la taille des réacteurs de décarboxylation.

Les contraintes de concentrations supérieures sont surtout imposées par des problèmes de solubilité ou viscosité des milieux réactionnels et sont donc dépendantes de la nature de l'acide aldonique soumis à la décarboxylation.

Cependant, d'une manière générale, on ne mettra pas en oeuvre de solutions aqueuses d'acides aldoniques d'une concentration supérieure à 500 g/l.

Dans le procédé de l'invention, le catalyseur de décarboxylation est constitué par des ions nickel qui peuvent être amenés sous forme d'un sel quelconque de nickel divalent.

Acétate, chlorure, nitrate de nickel par exemple conviennent parfaitement.

Dans le procédé de l'invention, on utilisera avantageusement les ions nickel sous forme de leur solution aqueuse de sels de nickel provenant par exemple de la régénération des échangeurs cationiques situés en aval de l'étape de décarboxylation, solution dans laquelle on pourra dissoudre ou diluer l'acide 2-cétoaldonique que l'on désire décarboxyler.

Des concentrations, exprimées en nickel, de 4 à 5 % par rapport à l'acide 2-cétoaldonique mis en oeuvre donnent de bons résultats dans le procédé de l'invention, tant en ce qui concerne le rendement que la pureté des cétoses obtenus.

La résine porteuse de groupements vinylpyridine utilisée dans le procédé de l'invention peut être de nature polyvinylpyridine ou encore posséder un squelette styrène-divinylbenzène.

De telles résines sont commercialisées sous la dénomination PVP 901 par la société IONAC par exemple ou sont connues sous la référence n° 100029 de la société PUROLITE INTERNATIONAL.

Des concentrations de 50 millilitres de résine par litre de solution d'acide 2-cétoaldonique mis en oeuvre donnent de bons résultats dans le procédé de l'invention lorsque celui-ci est mis en oeuvre de manière discontinue.

Dans un tel cas, la durée de réaction de décarboxylation est de l'ordre de une heure.

On peut, bien entendu, procéder aussi de manière continue en percolant les solutions d'acide 2-cétoaldonique au travers du lit de résine.

Dans ce cas, on préfère travailler avec un mouvement ascendant des solutions d'acides 2-cétoaldoniques au travers de la résine, et ce de manière à favoriser le dégagement du gaz carbonique qui se produit lors de la décarboxylation de l'acide cétoaldonique.

Cette façon de faire étant ainsi exposée, de simples opérations de routine permettront à l'homme de l'art de déterminer les débits et températures qui fournissent les meilleurs résultats en termes de productivité et de sélectivité de la réaction de décarboxylation.

D'une manière générale, que l'on procède de façon continue ou discontinue, on préfère mener la décarboxylation de l'acide 2-cétoaldonique jusqu'à ce qu'au moins 75 %, de manière préférée jusqu'à ce qu'au moins 85 % et de manière encore plus préférée, jusqu'à ce qu'au moins 90 % de l'acide 2-cétoaldonique se trouve décarboxylé pour fournir le cétose de rang immédiatement inférieur correspondant à cet acide.

Lorsque l'on met en oeuvre le procédé de l'invention d'une manière discontinue, la solution de cétose ainsi obtenue est simplement filtrée pour en ôter la résine. Cette résine ainsi filtrée est alors réemployée, sans qu'il soit nécessaire de la laver, pour une opération de décarboxylation ultérieure et peut ainsi être réutilisée un très grand nombre de fois sans que l'on constate de perte d'activité de cette résine.

Une telle opération de filtration n'est cependant pas nécessaire dans la mesure où l'on procède de façon continue à la décarboxylation des acides 2-cétoaldoniques.

Après cette opération de décarboxylation, les sirops de cétoses obtenus sont déminéralisés par exemple par électrodialyse ou par déminéralisation sur des échangeurs d'ions cationiques et anioniques régénérés respectivement sous forme hydrogène et sous forme hydroxyle.

Les électrodialysats ou les effluents de régénération des échangeurs cationiques, riches en sels de nickel ayant servi de catalyseur sont avantageusement récupérés pour y dissoudre à nouveau les acides 2-cétoaldoniques que l'on désire soumettre à décarboxylation.

Ainsi donc, le procédé de la présente invention permet d'obtenir avec d'excellents rendements et puretés des cétoses possédant un atome de carbone de moins que l'acide 2-cétoaldonique qui leur a donné naissance et ce, sans utilisation de solvants toxiques et dangereux.

Le procédé de la présente invention qui permet entre autres, d'obtenir du xylulose à partir de l'acide 2-cétogalactonique se rèvèle d'une particulière efficacité dans un procédé plus particulier d'obtention du xylitol à partir du lactose.

Un tel procédé plus particulier consiste à hydrolyser le lactose en glucose et galactose, à oxyder le galactose par voie chimique ou microbienne en acide 2-cétogalactonique, à décarboxyler cet acide en xylulose par le procédé de l'invention, puis à hydrogéner ce xylulose en xylitol, soit en l'état, soit de façon préférée après isomérisation enzymatique de ce xylulose en xylose ainsi qu'il est décrit par exemple dans le brevet US-A-5.096.820 dont la Demanderesse est cessionnaire.

Le procédé de la présente invention permet également d'obtenir l'érythrulose à partir de l'acide 2-cétoarabinonique. L'érythrulose ainsi obtenu peut ensuite être transformé par hydrogénation afin d'obtenir l'érythritol de façon avantageuse.

Avec le procédé selon l'invention, il est également possible d'obtenir le ribulose à partir de l'acide 2-cétogluconique. Le ribulose ainsi obtenu peut alors être transformé par hydrogénation en ribitol.

L'invention sera mieux comprise au moyen des exemples qui suivent et qui ont pour seul but de mieux illustrer l'invention sans vouloir la réduire aux modes de réalisation qui y sont décrits ou aux seuls acides 2-cétoaldoniques mis en oeuvre.

### EXEMPLE 1 :

Dans une cuve thermostatée et agitée, d'un volume total de 1,5 litre, on introduit 1 litre d'une solution d'acide 2-céto-D-gluconique.

On porte cette cuve à la température de 80°C puis on y ajoute 17 grammes de nitrate de nickel hexahydraté et 50 ml de résine PUROLITE n° 100029.

Cette résine est une résine anionique ayant le groupement fonctionnel vinylpyridine. Elle possède une capacité de 1,98 équivalent par litre sous forme de base libre, une porosité de 0,66 ml/gramme, possède un diamètre moyen de pores de 678 Angströms et une surface spécifique de 51,4 m² par gramme.

Elle se présente sous forme de billes dont 86,8 % ont un diamètre compris entre 425 et 1000 microns.

On laisse la réaction se dérouler pendant une heure puis on récupère le milieu réactionnel par simple filtration de la résine.

On récupère cette résine, et sans la laver, on la recycle dans une deuxième opération strictement identique puis à nouveau dans une troisième opération.

Au terme de ces trois essais on analyse le milieu réactionnel final. Celui-ci ne contient que du D-ribulose.

On a reporté les résultats obtenus dans le tableau qui suit où les concentrations de l'acide 2-céto-D-gluconique et du D-ribulose sont exprimées en moles par litre de milieu réactionnel filtré.

Le rendement de conversion en ribulose est lui aussi, molaire.

| | Essai 1 | Essai 2 | Essai 3 |
|---|---|---|---|
| Etat initial | | | |
| Acide 2-céto-D-gluconique | 0,386 | 0,386 | 0,386 |

| Etat final | | | |
|---|---|---|---|
| Acide 2-cédo-D-gluconique | 0 | 0 | 0 |
| D-ribulose | 0,13 | 0,29 | 0,34 |
| Conversion % | 100 | 100 | 100 |
| Rendement | 34,2 | 76,3 | 89,5 |

Ce tableau montre que la conversion de l'acide 2-céto-D-gluconique en D-ribulose est totale à chaque essai et que le rendement de la réaction augmente d'un essai à un autre.

Cette augmentation progressive du rendement est due à l'installation, elle aussi progressive, du régime permanent où il y aura équilibre entre la concentration du milieu extérieur et la concentration de la phase adsorbée dans les billes de résine.

On constate en effet que la succession de quelques autres essais amène rapidement le rendement à une valeur proche de 100 %.

Un ultime lavage de la résine permet d'en désorber le ribulose qu'elle a adsorbé et ainsi de retrouver pour la totalité des essais, un rendement proche de 100 % de décarboxylation de l'acide 2-céto-D-gluconique en D-ribulose.

L'hydrogénation catalytique de ce ribulose fournit un mélange équimoléculaire de D-ribitol et de D-arabitol.

### EXEMPLE 2 :

Dans une cuve thermostatée et agitée, on introduit 400 g d'eau dans lesquels on dissout 100 g (0,515 mole) d'acide 2-cétogalactonique et 8 g (0,0275 mole) de nitrate de nickel hexahydraté.

Puis on ajoute 625 ml de résine Purolite n° 100029 telle qu'utilisée dans l'exemple 1.

Sous agitation, on porte le mélange réactionnel à la température de 80°C pendant 40 minutes.

Après refroidissement on récupère le milieu réactionnel par filtration de la résine. On lave une fois à l'eau la résine. On mélange l'eau de lavage avec le milieu réactionnel. On analyse le milieu réactionnel. Celui-ci ne contient que du xylulose.

Le taux de conversion de l'acide 2-cétogalactonique est donc de 100 %. Le rendement molaire en xylulose est de 82,5 %.

### EXEMPLE 3 :

Dans une cuve thermostatée et agitée, on introduit 400 g d'eau dans lesquels on dissout 100 g (0,609 mole) d'acide 2-cétoarabinonique et 9,4 g (0,032 mole) de nitrate de nickel hexahydraté. Puis on ajoute 740 ml de résine Purolite n° 100029 telle qu'utilisée dans l'exemple 1.

Sous agitation on porte le mélange réactionnel à la température de 80°C pendant 60 minutes.

Après refroidissement on récupère le milieu réactionnel par filtration de la résine. On lave une fois à l'eau la résine. On mélange l'eau de lavage avec le milieu réactionnel. On analyse le milieu réactionnel. Celui-ci ne contient que de l'érythrulose.

Le taux de conversion de l'acide 2-cétoarabinonique est donc de 100 %. Le rendement molaire en érythrulose est de 40 %.

### Exemple 4 :

On recommence l'exemple 2 mais on porte le milieu réactionnel à 80 °C pendant 60 minutes au lieu de 40 minutes.

L'analyse du milieu réactionnel montre que le taux de conversion de l'acide 2-cétogluconique est de 100 %. Le rendement molaire en ribulose est de 95 %. La résine peut être réutilisée au moins 10 fois sans diminution de son activité et de sa sélectivité.

### Exemple 5 :

On fait passer en boucle sur une colonne thermostatée à 80°C contenant 125 ml de résine Purolite n° 100029 telle qu'utilisée dans l'exemple 1, une solution contenant, dans 400 g d'eau, 100 g (0,515 mole) d'acide 2-cétogluconique et 8 g (0,0275 mole) de nitrate de nickel hexahydraté.

L'alimentation est effectuée du bas vers le haut afin de faciliter l'élimination du dioxyde de carbone. La vitesse de passage dans la colonne est de 1V/V/h. On arrête la recirculation lorsque la conversion en acide 2-cétogluconique est totale. La durée totale de réaction est de 5 heures et le rendement molaire en ribulose est de 70 %.

## Revendications

1. Procédé de décarboxylation catalytique des acides 2-cétoaldoniques par les ions nickel caractérisé par le fait qu'une solution aqueuse d'un acide 2-cétoaldonique est mise au contact d'une résine porteuse de groupements vinylpyridine.

2. Procédé de décarboxylation catalytique des acides 2-cétoaldoniques par les ions nickel selon la revendication 1, caractérisé par le fait que la décarboxylation est effectuée à une température de 40 à 100°C, et de préférence de 70 à 90°C.

3. Procédé de décarboxylation catalytique des acides 2-cétoaldoniques par les ions nickel selon les revendications 1 ou 2, caractérisé par le fait que la solution aqueuse de l'acide aldonique est d'une concentration comprise entre 50 et 500 g/l.

4. Procédé de décarboxylation catalytique des acides 2-cétoaldoniques par les ions nickel selon l'une quelconque des revendications 1 à 3, caractérisé par le fait que les solutions de cétoses obtenues sont déminéralisées sur des échangeurs d'ions cationiques et anioniques régénérées respectivement sous forme hydrogène et sous forme hydroxyle.

5. Procédé de décarboxylation catalytique des acides 2-cétoaldoniques par les ions nickel selon la revendication 4, caractérisé par le fait que ces ions nickel proviennent de la régénération des échangeurs d'ions cationiques.

6. Procédé de décarboxylation catalytique d'un acide 2-cétoaldonique en xylulose selon l'une quelconque des revendications 1 à 5, caractérisé par le fait que l'acide 2-cétoaldonique est l'acide 2-cétogalactonique.

7. Procédé de fabrication du xylitol par hydrogénation du xylulose caractérisé par le fait que le xylulose est obtenu par le procédé selon la revendication 6.

8. Procédé de décarboxylation catalytique d'un acide 2-cétoaldonique en ribulose selon l'une quelconque des revendications 1 à 5 caractérisé par le fait que l'acide 2-cétoaldonique est l'acide 2-cétogluconique.

9. Procédé de fabrication du ribitol par hydrogénation du ribulose caractérisé par le fait que le ribulose est obtenu par le procédé selon la revendication 8.

10. Procédé de décarboxylation catalytique d'un acide 2-cétoaldonique en érythrulose selon l'une quelconque des revendications 1 à 5, caractérisé par le fait que l'acide 2-cétoaldonique est l'acide 2-cétoarabinonique.

11. Procédé de fabrication de l'érythritol par hydrogénation de l'érythrulose, caractérisé par le fait que l'érythrulose est obtenu par le procédé selon la revendication 10.

## Patentansprüche

1. Verfahren zur katalytischen Decarboxylierung von 2-Ketoaldonsäuren durch Nickelionen, dadurch gekennzeichnet, dass eine wässrige Lösung einer 2-Ketoaldonsäure mit einem Vinylpyridingruppen tragenden Harz in Kontakt gebracht wird.

2. Verfahren zur katalytischen Decarboxylierung von 2-Ketoaldonsäuren durch Nickelionen nach Anspruch 1, dadurch gekennzeichnet, dass die Decarboxylierung bei einer Temperatur von 40 bis 100°C und vorzugsweise von 70 bis 90°C durchgeführt wird.

3. Verfahren zur katalytischen Decarboxylierung von 2-Ketoaldonsäuren durch Nickelionen nach den Ansprüchen 1 oder 2, dadurch gekennzeichnet, dass die wässrige Lösung der Aldonsäure eine Konzentration zwischen 50 und 500 g/l besitzt.

4. Verfahren zur katalytischen Decarboxylierung von 2-Ketoaldonsäuren durch Nickelionen nach einem der Ansprüche 1 bis 3, dadurch gekennzeichnet, dass die erhaltenen Ketosenlösungen auf regenerierten kationischen und anionischen Ionenaus-tauschern in Wasserstoff- bzw. Hydroxylform demineralisiert werden.

5. Verfahren zur katalytischen Decarboxylierung von 2-Ketoaldonsäuren durch Nickelionen nach Anspruch 4, dadurch gekennzeichnet, dass die Nickelionen von der Regeneration der kationischen Ionenaustauscher stammen.

6. Verfahren zur katalytischen Decarboxylierung einer 2-Keto-aldonsäure zu Xylulose nach einem der Ansprüche 1 bis 5, dadurch gekennzeichnet, dass die 2-Ketoaldonsäure 2-Ketogalactonsäure ist.

7. Verfahren zur Herstellung von Xylitol durch Hydrierung von Xylulose, dadurch gekennzeichnet, dass die Xylulose in dem Verfahren nach Anspruch 6 erhalten wird.

8. Verfahren zur katalytischen Decarboxylierung einer 2-Ketoaldonsäure zu Ribulose nach einem der Ansprüche 1 bis 5, dadurch gekennzeichnet, dass die 2-Ketoaldonsäure 2-Ketoglucon-säure ist.

9. Verfahren zur Herstellung von Ribitol durch Hydrierung von Ribulose, dadurch gekennzeichnet, dass die Ribulose in dem Verfahren nach Anspruch 8 erhalten wird.

10. Verfahren zur katalytischen Decarboxylierung einer 2-Ketoaldonsäure zu Erythrulose nach einem der Ansprüche 1 bis 5, dadurch gekennzeichnet, dass die 2-Ketoaldonsäure 2-Ketoarabinonsäure ist.

11. Vefahren zur Herstellung von Erythritol durch Hydrierung von Erythrulose, dadurch gekennzeichnet, dass die Erythrulose in dem Verfahren nach Anspruch 10 erhalten wird.

## Claims

1. Catalytic decarboxylation process for 2-ketoaldonic acids by nickel ions wherein an aqueous solution a 2-ketoaldonic acid is put in contact with a resin carrying vinylpyridine groups.

2. Catalytic decarboxylation process for 2-ketoaldonic acids by nickel ions according to Claim 1, wherein the decarboxylation is carried out at a temperature from 40 to 100°C, preferably from 70 to 90°C.

3. Catalytic decarboxylation process for 2-ketoaldonic acids by nickel ions according to any one of Claims 1 or 2, wherein the aqueous solution of aldonic acid is at a concentration comprised between 50 and 500 g/l.

4. Catalytic decarboxylation process for 2-ketoaldonic acids by nickel ions according to any one of Claims 1 to 3, wherein the solutions of ketoses obtained are demineralized on cationic and anionic ion exchangers regenerated in the form of hydrogen and in the form of hydroxyl respectively.

5. Catalytic decarboxylation process for 2-ketoaldonic acids by nickel ions according to Claim 4, wherein the nickel ions are produced by regeneration from cationic ion exchangers.

6. Catalytic decarboxylation process for a 2-ketoaldonic acid into xylulose according to any one of Claims 1 to 5, wherein the 2-ketoaldonic acid is 2-ketogalactonic acid.

7. Production process for xylitol by hydrogenation of xylulose wherein the xylulose is obtained by the process according to Claim 6.

8. Catalytic decarboxylation process for a 2-ketoaldonic acid into ribulose according to any one of Claims 1 to 5, wherein the 2-ketoaldonic acid is a 2-ketogluconic acid.

9. Production process for ribitol by hydrogenation of ribulose wherein the ribulose is obtained by the process according to Claim 8.

10. Catalytic decarboxylation process for a 2-ketoaldonic acid into erythrulose according to any one of claims 1 to 5, wherein the 2-ketoaldonic acid is 2-ketoarabinonic acid.

11. Production process for erythritol by hydrogenation of erythrulose, wherein the erythrulose is obtained by the process according to Claim 10.
